(19) Europäisches Patentamt
European Patent Office
Office européen des brevets

(11) Publication number: **0 194 752**
**A1**

(12) # EUROPEAN PATENT APPLICATION

(21) Application number: 86300887.6

(22) Date of filing: 10.02.86

(51) Int. Cl.[4]: C 07 D 211/90
C 07 D 401/12, C 07 D 413/04
A 61 K 31/44

(30) Priority: 11.02.85 GB 8503428

(43) Date of publication of application:
17.09.86 Bulletin 86/38

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Hargreaves, Rodney Brian
42 Deva Close
Poynton Cheshire(GB)

(72) Inventor: McLoughlin, Bernard Joseph
7 Pexhill Drive
Macclesfield Cheshire(GB)

(72) Inventor: Mills, Stuart Dennett
17 Harrington Drive
Gawsworth Macclesfield, Sk11 9RD(GB)

(74) Representative: Slatcher, Reginald Peter et al,
Imperial Chemical Industries PLC Legal Department:
Patents P.O. Box 6 Bessemer Road
Welwyn Garden City AL7 1HD(GB)

(54) **Dihydropyridine alkanol amines, process for their preparation and pharmaceutical compositions containing them.**

(57) A dihydropyridine of the formula:-

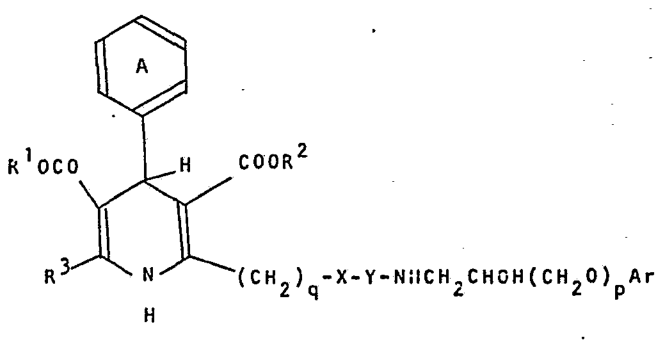

wherein $R^1$ and $R^2$ each is alkyl or alkoxyalkyl,

wherein $R^3$ is alkyl, wherein benzene ring A bears one or more substituents selected from halogeno, cyano, nitro, trifluoromethyl and alkyl or bears the substituent =N–O–N= attached to the 2- and 3-positions;

wherein Ar is phenyl, naphthul, tetrahydronaphthyl, indanyl or indenyl which is unsubstituted or which bears one or more substituents

or Ar is a 5- or 6-membered heterocyclic ring containing 1, 2 or 3 heteroatoms selected from oxygen, nitrogen and sulphur, which ring is saturated or unsaturated, and which ring is unsubstituted or bears one or more substituents, wherein p is 0 or 1;

wherein q is 1, 2, 3 or 4;

wherein X is –O–, –NH–, –NHCO– or –CONH–;

and wherein Y is straight-or branched-chain alkylene which may optionally be interrupted by one or two groups selected from oxygen, sulphur, imino, substituted imino, phenylene, substituted phenylene, pyridylene, cycloalkylene, 1,4-piperazinediyl, 1,4-piperidinediyl and amido groups; or an acid-addition salt thereof, processes for their manufacture and pharmaceutical compositions containing them. The compounds possess either beta-adrenergic blocking or calcium ion slow channel blocking properties or both such properties, and may be used in the treatment of hypertension.

EP 0 194 752 A1

## NITROGEN-CONTAINING COMPOUNDS

This invention relates to new nitrogen-containing compounds and more particularly it relates to new dihydropyridine derivatives which possess antihypertensive properties.

Many 2,6-dialkyl-4-aryl-1,4-dihydropyridine-3,5-dicarboxylate derivatives are known which inhibit the movement of calcium ions in the cardiovascular system of warm-blooded animals, and which thereby produce an antihypertensive effect. The most-used of these is nifedipine, which is dimethyl 1,4-dihydro-2,6-dimethyl-4-*o*-nitrophenylpyridine-3,5-dicarboxylate.

Also known are many 1-aryloxy-3-amino-propan-2-ol derivatives which possess beta-adrenergic receptor blocking properties and which also produce an antihypertensive effect. Two of the most-used of these are propranolol and atenolol, which are respectively 1-(naphth-1-yloxy)- and 1-*p*-carbamoylmethylphenoxy-3-isopropylaminopropan-2-ol.

The only described attempt to combine these two types of chemical structure into one molecule is reported by Merck workers in the Journal of Medicinal Chemistry, 1981, Vol. 24, pages 628 to 631, in which a 3-amino-2-hydroxypropoxy substituent was introduced into the 4-aryl substituent of a 4-aryl-1,4-dihydropyridine derivative, without much success in producing a compound with antihypertensive activity of the type sought by the authors.

We have now found that compounds which do possess useful antihypertensive activity may be obtained by suitably combining a 3-aryloxy-2-hydroxypropylamino moiety with a 1,4-dihydropyridine moiety.

According to the present invention there is provided a dihydropyridine of the formula:

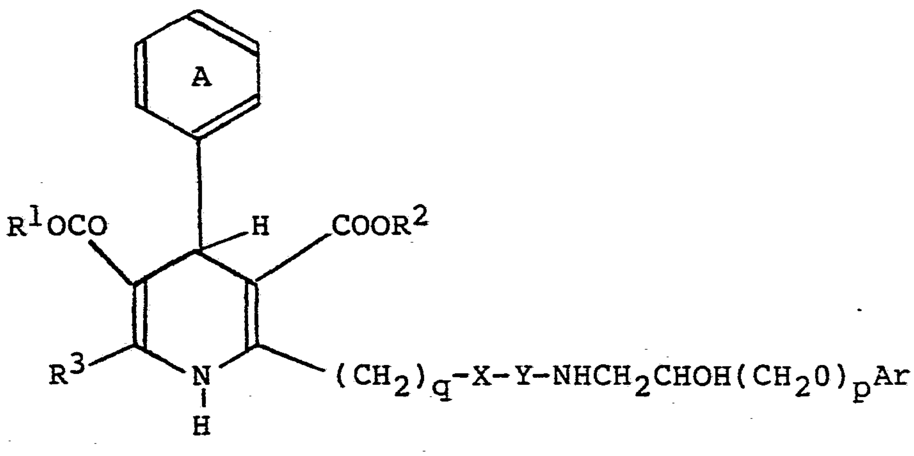

wherein $R^1$ and $R^2$, which may be the same or different, each is alkyl or alkoxyalkyl each of up to 6 carbon atoms:
wherein $R^3$ is alkyl of up to 6 carbon atoms;
wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus);
wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
or Ar is a 5- or 6-membered heterocyclic ring containing 1, 2 or 3 heteroatoms selected from oxygen, nitrogen and sulphur, which ring is saturated or unsaturated, which ring is unsubstituted or bears one or more substituents selected from oxo, halogeno, trifluoromethyl, phenyl and morpholino, and alkyl and alkoxy each of up to 6 carbon atoms, and arylalkyl of up to 12 carbon atoms, and which

ring may also be fused to a benzene ring, Ar being joined to the rest of the molecule either from the heterocyclic ring or from the fused benzene ring;
wherein p is 0 or 1;
wherein q is 1, 2, 3 or 4;
wherein X is -O-, -NH-, -NHCO- or -CONH-;
and wherein Y is straight-or branched-chain alkylene of 2 to 12 carbon atoms which may optionally be interrupted by one or two groups selected from oxygen (-O-), sulphur (-S-), imino and substituted imino ($-NR^4$ wherein $R^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof.

It will be observed that the dihydropyridine derivative of the invention possesses at least two asymmetric carbon atoms, namely the carbon atom of the -CHOH- group in the alkanolamine chain, and the carbon atom at the 4-position of the dihydropyridine nucleus, and it can therefore exist in racemic and optically-active forms. It is to be understood that this invention encompasses the racemic form of the dihydropyridine derivative and any optically-active form which possesses antihypertensive activity, it being a matter of common general knowledge how a racemic compound may be resolved into optically-active forms, and how the antihypertensive activity of these forms may be determined. It is further to be understood that beta-adrenergic blocking activity usually predominates in that optically-active form which has the "S" absolute configuration of the said -CHOH- group in the alkanolamine chain when p is 1 and the "R" absolute configuration when p is 0.

A suitable value for $R^1$, $R^2$, $R^3$, $R^4$ or a substituent in benzene ring A or Ar when it is alkyl is, for example, methyl, ethyl or isopropyl.

A suitable value for $R^1$ or $R^2$ when it is alkoxyalkyl is, for example, methoxyethyl, ethoxyethyl or propoxyethyl.

A suitable halogeno substituent in the benzene ring A or in Ar is, for example fluoro, chloro or bromo.

A suitable value for the alkenyl, alkoxy, alkenyloxy, alkoxyalkoxy, alkylthio, carbamoylalkyl or alkanoylamino substituent in Ar is, for example, allyl, methoxy, ethoxy, isopropoxy, allyloxy, methoxyethoxy, methylthio, carbamoylmethyl or acetamido.

A suitable value for $R^4$ when it is alkanoyl, or for an alkanoyl substituent in Ar is, for example,formyl, acetyl or benzoyl.

A suitable value for $R^4$ when it is aralkyl, or for an aralkyl substituent in Ar is, for example, benzyl.

A suitable value for Ar when it is heterocyclic, either single ring or benzo-fused ring, is, for example, 4-morpholino-1,2,5-thiadiazol-3-yl, 1-methyl-4-indolyl, 2-oxo-1,2,3,4-tetrahydro-5-quinolyl, 4-indolyl, 4-carbazolyl, 4-benzo[b]thienyl, 5-benzo[1,4]dioxanyl or 3-cyano-2-pyridyl.

A suitable value for Y is, for example, straight-chain alkylene of the formula $(-CH_2)_n-$, wherein n is an integer from 2 to 12;

or $-(CH_2)_mC(CH_3)_2-$;

or $-(CH_2)_m-NH-(CH_2)_n-$

$-(CH_2)_m-N(CH_3)-(CH_2)_n-$

$-(CH_2)_m-O-(CH_2)_n-$

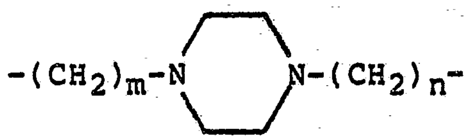

$-(CH_2)_m-CONH-(CH_2)_n-$

wherein m and n, which may be the same or different, each is 2,3,4 or 5;

or 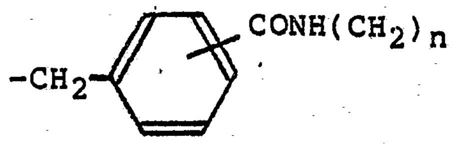

$-CH_2CONH(CH_2)_n-$

wherein n is 2, 3, 4 or 5;

or 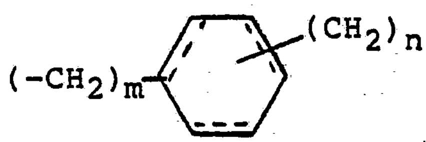 or 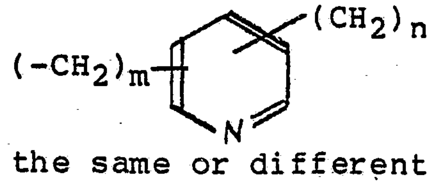

and wherein m and n, which may be the same or different, each is 1, 2, 3 or 4 and wherein the double bonds in the carbocyclic ring are optional (that is, cyclohexylene- or phenylene- bis-alkylene).

A preferred dihydropyridine derivative of the invention has the formula stated above wherein $R^1$ and $R^2$, which may be the same or different, each is methyl or ethyl, wherein $R^3$ is methyl, wherein ring A is 2-chlorophenyl or 3-nitrophenyl, wherein Ar is phenyl which is unsubstituted or bears a chloro, cyano, methyl or methoxy substituent at the 2-position, or Ar is unsubstituted 1-naphthyl, 4-indolyl or 4-carbazolyl, wherein p and q are both 1, wherein X is -O-, -NHCO- or -CONH- and wherein Y is alkylene of 2 to 5 carbon atoms which may be interrupted by -O-, -NH-or -CONH-, or is an acid-addition salt of such a compound.

Preferably $R^1$ is methyl or ethyl, $R^2$ is ethyl, X is -O- and Y is alkylene of 2 to 4 carbon atoms which may be interrupted by -O-.

A suitable acid-addition salt of a dihydropyridine derivative of the invention is, for example, a salt derived from an inorganic acid, for example a hydrochloride, hydrobromide, phosphate or sulphate, or a salt derived from an organic acid, for example an oxalate, lactate, succinate, tartrate, acetate, salicylate, citrate, benzoate, beta-naphthoate or adipate.

Specific dihydropyridine derivatives of the invention are hereinafter described in the Examples. Of these, preferred compounds are:-

ethyl 2-[2-(3-o-cyanophenoxy-2-hydroxypropylamino)ethoxy]methyl-1,4-dihydro-5-methoxycarbonyl-6-methyl-4-m-nitrophenylpyridine-3-carboxylate;

ethyl 4-o-chlorophenyl-2-[2-(3-o-cyanophenoxy-2-hydroxypropylamino)ethoxy]methyl-1,4-dihydro-5-methoxycarbonyl-6-methylpyridine-3-carboxylate;

ethyl 2-{2-[3-(carbazol-4-yloxy)-2-hydroxypropylamino]ethoxy}methyl-1,4-dihydro-5-methoxycarbonyl-6-methyl-4-m-nitrophenylpyridine-3-carboxylate;

diethyl 2-[2-(3-o-cyanophenoxy-2-hydroxypropylamino)ethoxy]methyl-1,4-dihydro-6-methyl-4-m-nitrophenylpyridine-3,5-dicarboxylate;

ethyl 2-[3-(3-o-cyanophenoxy-2-hydroxypropylamino)propoxy]methyl-1,4-dihydro-5-methoxycarbonyl-6-methyl-4-m-nitrophenylpyridine-3-carboxylate;

ethyl 2-[4-(3-o-cyanophenoxy-2-hydroxypropylamino)-butoxy]methyl-1,4-dihydro-5-methoxycarbonyl-6-methyl-4-m-nitrophenylpyridine-3-carboxylate; and

ethyl 2-{2-[2-(3-o-cyanophenoxy-2-hydroxypropylamino)-ethoxy]ethoxy}methyl-1,4-dihydro-5-methoxycarbonyl-6-methyl-4-m-nitrophenylpyridine-3-carboxylate.

The dihydropyridine derivatives of the invention may be manufactured by any chemical process known to be useful for the manufacture of chemically-analogous compounds.

One preferred process for the manufacture of a dihydropyridine derivative of the invention comprises the reaction of an amine of the formula:-

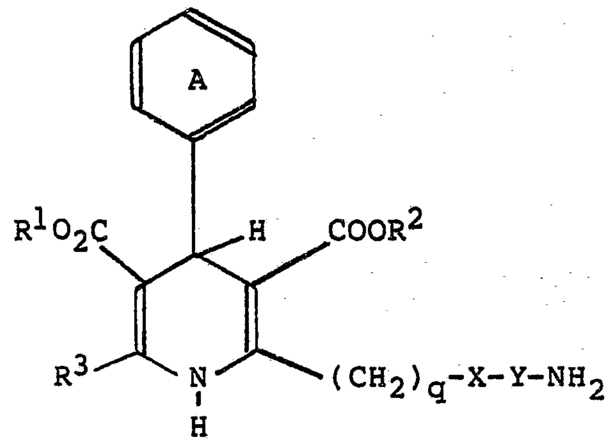

wherein A, $R^1$, $R^2$, $R^3$, q, X and Y have the meanings stated above, with an epoxide of the formula:-

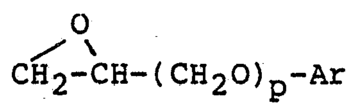

wherein Ar and p have the meanings stated above, or, when p is 0, with a haloketone of the formula

$$HalCH_2CO\text{-}Ar$$

wherein Ar has the meaning stated above and wherein Hal stands for a halogeno group, for example bromo, followed by reduction, for example with sodium borohydride, of the aminoketone thus obtained.

The reaction may be carried out in an alcoholic diluent or solvent, for example in isopropanol, at a temperature of up to the boiling point of said diluent or solvent.

A second preferred process for the manufacture of a dihydropyridine derivative of the invention wherein the group -Y- is alkylene interrupted as stated above comprises joining the two parts of the molecule at the point of interruption of Y. Thus, for example, when Y is alkylene interrupted by an amido group -CONH-, the process comprises the reaction of an acid of the formula:-

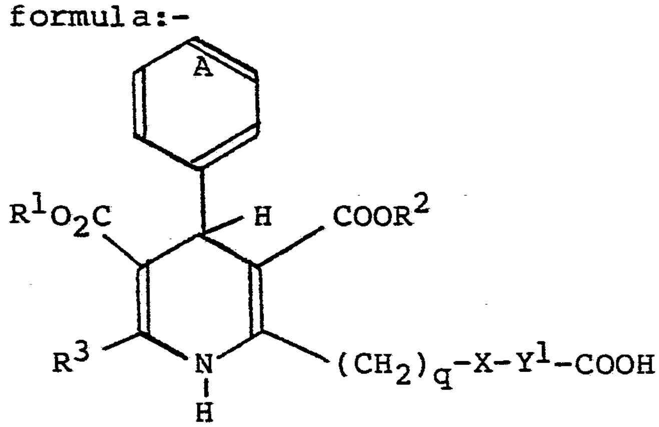

wherein A, $R^1$, $R^2$, $R^3$, q and X have the meanings stated above, or an activated derivative thereof, with an amine of the formula:-

$$H_2N\text{-}Y^2\text{-}NHCH_2CHOH(CH_2O)_pAr$$

wherein Ar and p have the meanings stated above, and wherein $Y^1$ and $Y^2$ are such that $-Y^1\text{-}CONH\text{-}Y^2-$ has the same meaning as stated above for Y.

A third process for the manufacture of a dihydropyridine derivative of the invention comprises the reaction of a compound of the formula:-

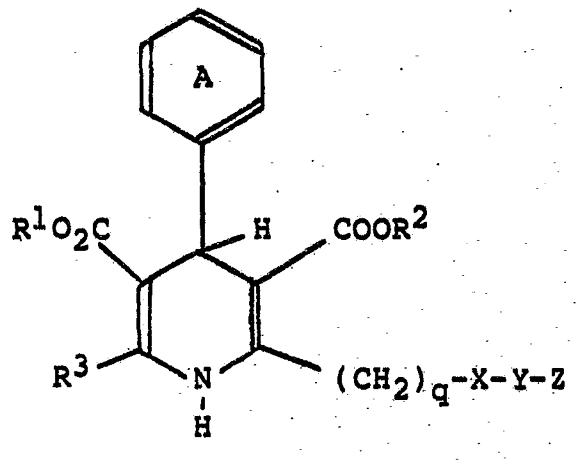

wherein A, $R^1$, $R^2$, $R^3$, q, X and Y have the meanings stated above and wherein Z stands for a displaceable group, with an amine of the formula:-

$$H_2NCH_2CHOH(CH_2O)_p-Ar$$

wherein Ar and p have the meanings stated above.

A suitable value for Z is, for example, a halogeno group, for example a bromo or chloro group, or a sulphonyloxy group, for example a methanesulphonyloxy or *p*-toluenesulphonyloxy group.

A fourth process for the manufacture of a dihydropyridine derivative of the invention comprises the reaction of an aldehyde of the formula

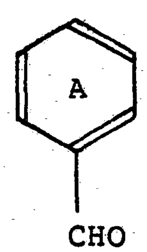

wherein A has the meaning stated above, an aminocrotonate of the formula

0194752

$$R^3-\underset{}{\overset{NH_2}{\overset{|}{C}}}=CH-CO_2R^1$$

wherein $R^1$ and $R^3$ have the meanings stated above and a ketoacid derivative of the formula

$$R^2O_2CCH_2CO(CH_2)_q-X-Y-NHCH_2CHOH(CH_2O)_p-Ar$$

wherein Ar, p, q, $R^2$, X and Y have the meanings stated above.

This process may be carried out in a diluent or solvent at an elevated temperature, conditions conventionally used for the Hantszch synthesis of dihydropyridines.

As stated above, the dihydropyridine derivatives of the invention possess antihypertensive activity. This may be demonstrated by the ability of the compound to reduce the blood pressure of a spontaneously hypertensive rat, or of a rat made hypertensive by treatment with deoxycorticosterone acetate, or of a dog made hypertensive by the Goldblatt technique of unilateral nephrectomy and clipping of the contralateral kidney. These are all standard tests used to demonstrate antihypertensive effects of medicaments.

Some of the dihydropyridine derivatives of the invention possess beta-adrenergic blocking properties, some of them possess calcium ion slow-channel blocking properties and some of them possess both such activities. A preferred dihydropyridine derivative of the invention possesses both such activities. Beta-adrenergic blocking activity may be demonstrated in vivo by the ability of the compound to inhibit isoprenaline-induced tachycardia in a rat or cat, or in vitro by shifting to the right the dose- response curve

of a guinea pig atrium to isoprenaline. Calcium ion slow channel blocking activity may be demonstrated _in vitro_ by the ability of the compound to reduce spontaneous contraction in a rat portal vein preparation. These also are all standard tests used to demonstrate the stated activities.

Because of the beta-adrenergic blocking and/or calcium slow channel blocking properties a dihydropyridine of the invention may also be useful in the treatment of heart diseases such as angina pectoris and cardiac arrhythmias.

At doses of a dihydropyridine derivative which produce effective antihypertensive activity in a rat or dog no symptom of toxicity is apparent.

The dihydropyridine derivative of the invention may be administered to warm-blooded animals, including man, in the form of a pharmaceutical composition comprising as active ingredient at least one dihydropyridine derivative of the invention, or an acid-addition salt thereof, in association with a pharmaceutically-acceptable diluent or carrier therefor.

A suitable composition is, for example, a tablet, capsule, aqueous or oily solution or suspension, dispersible powder, spray or aerosol formulation.

The pharmaceutical composition may contain, in addition to the dihydropyridine derivative of the invention, one or more drugs selected from sedatives, for example phenobarbitone, meprobamate, chloropromazine and the benzodiazepine sedative drugs, for example chlordiazepoxide and diazepam; vasodilators, for example glyceryl trinitrate, pentaerythritol tetranitrate, isosorbide dinitrate and hydralazine; diuretics, for example chlorthalidone, bendrofluazide, hydrochlorothiazide and chlorothiazide; other antihypertensive agents, for example reserpine, bethanidine and guanethidine; cardiac membrane stabilising agents, for example quinidine; agents used in the treatment of Parkinson's disease and other tremors, for example benzhexol; cardiotonic agents, for example digitalis preparations; and alpha-adrenergic blocking agents, for example phentolamine.

When used for the treatment of heart diseases, for example angina pectoris and cardiac arrhythmias, or for the treatment of hypertension in man, it is expected that the dihydropyridine derivative would be given to man at a total oral dose of between 20 mg. and 600 mg. daily, or at an intravenous dose of between 1 mg. and 20 mg.

Preferred oral dosage forms are tablets or capsules containing between 10 and 100 mg., and preferably 10 mg. or 50 mg., of active ingredient. Preferred intravenous dosage forms are sterile solutions of the dihydropyridine derivative or of a non- toxic acid-addition salt thereof, containing between 0.05% and 1% w/v of active ingredient, and more particularly containing 0.1% w/v of active ingredient.

The invention is illustrated but not limited by the following Examples:-

Example 1

A mixture of ethyl 2-(2-aminoethoxy)methyl-1,4-dihydro-5-methoxycarbonyl-6-methyl-4-*m*-nitrophenyl-pyridine-3-carboxylate (isolated from 1.1. g. of the oxalate salt, described in European Specification No. 89167, by shaking with aqueous 10% sodium carbonate solution and extracting into methylene chloride), 1-*o*-cyanophenoxy-2,3-epoxypropane (0.35 g.) and toluene (15 ml.) was heated at 100°C. for 5 hours, and then evaporated to dryness, and the residue was purified by flash chromatography on a silica gel column (Merck 9385)

using a 19:1 v/v mixture of methylene chloride and methanol as eluant. The product was dissolved in ethanol, a saturated solution of oxalic acid in ethanol was added and the mixture was filtered. There was thus obtained as solid product ethyl 2-[2-(3-*o*-cyanophenoxy-2-hydroxypropylamino)ethoxy]methyl-1,4-dihydro-5-methoxycarbonyl-6-methyl-4-*m*-nitrophenylpyridine-3-carboxylate oxalate, m.p. 121-125°C.

The process described above was repeated using phenyl glycidyl ether in place of the corresponding *o* cyanophenoxy compound, and there was thus obtained as an oil ethyl 1,4-dihydro-2-[2-(2-hydroxy-3-phenoxypropylamino)ethoxy]methyl-5-methoxycarbonyl-6-methyl-4-*m*-nitrophenylpyridine-3-carboxylate oxalate, the structure of which was confirmed by elemental analysis and proton magnetic resonance spectroscopy.

The process described above was repeated using the corresponding 4-*o*-chlorophenylpyridine-3-carboxylate in place of the 4-*m*-nitrophenyl compound, and omitting the oxalate salt formation step. There was thus obtained as an oil ethyl 4-*o*-chlorophenyl-2-[2-(3-*o*-cyanophenoxy-2-hydroxypropylamino)ethoxy]methyl-1,4-dihydro-5-methoxycarbonyl-6-methylpyridine-3-carboxylate, the structure of which was confirmed by elemental analysis and proton magnetic resonance spectroscopy.

**Example 2**

The process described in Example 1 was repeated using the appropriate alkyl 2-aminoalkoxymethylpyridine-3-carboxylate (prepared as described in United Kingdom Specification No. 1585978) and the appropriate epoxide as starting materials, and omitting the oxalate formation step. There were thus obtained as oils the compounds described in the following table, the structures of all of which were confirmed by elemental

analysis, mass spectroscopy and proton magnetic resonance spectroscopy:-

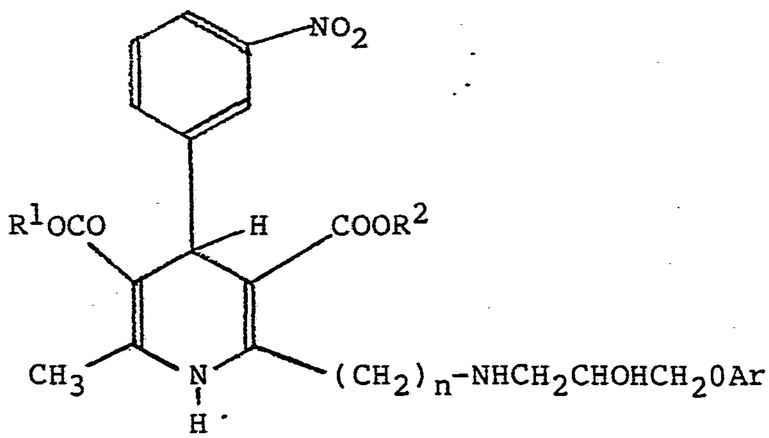

| $R^1$ | $R^2$ | n | Ar | hydrate |
|---|---|---|---|---|
| ethyl | ethyl | 2 | 2-cyanophenyl | - * |
| methyl | ethyl | 2 | 2-methoxyphenyl | mono |
| methyl | ethyl | 2 | 4-indolyl | hemi * |
| methyl | ethyl | 2 | 4-carbazolyl | hemi |
| methyl | ethyl | 3 | phenyl | - |

* The reaction mixture was heated for 18 hours.

**Example 3**

A mixture of ethyl 1,4-dihydro-2-[2-(2-mesyloxyethoxy)ethoxymethyl]-5-methoxycarbonyl-6-methyl-4-*m*-nitrophenylpyridine-3-carboxylate (0.94 g.), 1-amino-3-*o*-cyanophenoxypropan-2-ol (0.67 g.) and dimethylformamide (10 ml.) was heated at 100°C. for 4 hours and then evaporated to dryness. The residue was dissolved in methylene chloride (20 ml.) and the

solution was washed with water, dried and evaporated to dryness. The residue was partially purified by flash chromatography on a silica gel (Merck 9385) column using as eluent a 60:35:20 v/v/v mixture of toluene, ethanol and ethyl acetate to which was added an increasing amount of aqueous ammonia solution (specific gravity 0.88) up to a 60:35:20:1 v/v/v/v mixture. The product was finally purified by preparative thin-layer chromatography on silica gel plates (20 cm x 20 cm; 2 mm thick) using a 99:1 v/v mixture of ethyl acetate and triethylamine as developing solvent. There was thus obtained as an oil ethyl 2-{2-[2-(3-*o*-cyanophenoxy-2-hydroxypropylamino)ethoxy]ethoxymethyl}-1,4-dihydro-5-methoxycarbonyl-6-methyl-4-*m*-nitrophenylpyridine-3-carboxylate hemihydrate, the structure of which was confirmed by elemental analysis, mass spectroscopy and proton magnetic resonance spectroscopy.

The mesyloxy compound used as starting material was prepared as follows:-

A mixture of 3-oxapentamethylene-1,5-diol (10 g.), triphenylmethyl chloride (trityl chloride, 8.8 g.) and pyridine (100 ml.) was heated at 100°C. for 2 hours, diluted with water (100 ml.) and extracted with diethyl ether. The ethereal extract was washed successively with water, aqueous N-hydrochloric acid, water, 10% w/v aqueous sodium carbonate solution and water, dried and evaporated to dryness. The residue was crystallised from a mixture of toluene and petroleum ether (b.p. 60-80°C.) and there was thus obtained 2-(2-trityloxyethoxy)ethanol, m.p. 113-114°C.

A solution of the above ethanol (6.3 g.) in tetrahydrofuran (30 ml.) was added dropwise during 5 minutes to a stirred suspension of sodium hydride (1.9 g. of a 50% w/w suspension in paraffin oil from which the oil had been washed with petroleum ether) in

tetrahydrofuran (10 ml.) and the mixture was stirred at laboratory temperature for 4.5 hours and then cooled to 0°C. Ethyl 4-chloro-3-oxobutyrate (2.98 g.) was added dropwise during 30 minutes and the mixture was allowed to warm up to laboratory temperature and was stirred for 18 hours. Ethanol (10 ml.) was added, the solution was poured into aqueous N-hydrochloric acid (30 ml.) and the mixture was extracted three times with diethyl ether (50 ml. each time). The combined ethereal extracts were washed with 10% w/v aqueous sodium carbonate solution and then with water, dried and evaporated to dryness.

A mixture of the ethyl 3-oxo-4-[2-(2-trityloxyethoxy)ethoxy]butyrate thus obtained as an oil (2.4 g.), methyl 3-aminocrotonate (0.58 g.), 3-nitrobenzaldehyde (0.76 g.) and ethanol (15 ml.) was heated under reflux for 18 hours and then evaporated to dryness, and the product was purified by flash chromatography on a silica gel (Merck 9385) column using a 4:1 v/v mixture of petroleum ether (b.p. 60-80°C.) and ethyl acetate as eluent. There was thus obtained ethyl 1,4-dihydro-2-[2-(2-trityloxyethoxy)ethoxymethyl]-5-methoxycarbonyl-6-methyl-4-*m*-nitrophenylpyridine-3-carboxylate.

A solution of the above compound (3.8 g.) in methylene chloride (10 ml.) was added dropwise to a stirred suspension of zinc bromide (6.0 g.) in methylene chloride (15 ml.) containing ethanol (0.5 ml.), the mixture was stirred at laboratory temperature for 5 minutes and sufficient ethanol was added to produce a clear solution. The mixture was evaporated to dryness, ethyl acetate and water were added and the mixture was filtered through a filter-aid. The organic layer of the filtrate was separated, washed with aqueous 10% w/v sodium carbonate solution and then with saturated aqueous sodium chloride solution, dried and evaporated

to dryness. The residue was purified by flash chromatography on a silica gel (Merck 9385) column using ethyl acetate as eluent and there was thus obtained as an oil ethyl 1,4-dihydro-2-[2-(2-hydroxyethoxy)ethoxymethyl]-5-methoxycarbonyl-6-methyl-4-m-nitrophenylpyridine-3-carboxylate.

Methanesulphonyl chloride (0.17 ml.) was added dropwise to a stirred solution of the above compound (1.0 g.) and triethylamine (0.22 g.) in methylene chloride (20 ml.) at -4°C. and the mixture was stirred at 0°C. for 2 hours, washed with aqueous 10% w/v sodium carbonate solution, dried and evaporated to dryness. There was thus obtained the desired mesyloxy compound which was used without further purification.

Example 4

The process described in Example 3 was repeated using ethyl 1,4-dihydro-2-(4-mesyloxybutyloxy-methyl)-5-methoxycarbonyl-6-methyl-4-m-nitrophenylpyridine-3-carboxylate as starting material. There was thus obtained as an oil ethyl 2-[4-(4-o-cyanophenoxy-2-hydroxypropylamino)butyloxymethyl]-1,4-dihydro-5-methoxycarbonyl-6-methyl-4-m-nitrophenylpyridine-3-carboxylate dihydrate, the structure of which was confirmed by elemental analysis, mass spectroscopy and proton magnetic resonance spectroscopy.

The mesyloxy compound used as starting material was obtained by a similar process for that described in the second part of Example 3. except that tetramethylene-1,4-diol was used as initial intermediate for reaction with ethyl 4-chloro-3-oxobutyrate.

Example 5

1-(2-Aminoethylamino)-3-phenoxypropan-2-ol (0.16 g.) was added to a stirred mixture of N-(4-o-chlorophenyl-3,5-diethoxycarbonyl-1,4-dihydro-6-

methylpyrid-2-ylmethyl)-2-carbamoylacetic acid (0.3 g.), carbonyldiimidazole (0.115 g.) and dimethylformamide (10 ml.) which had been stirred at laboratory temperature for 2 hours, and the mixture was stirred at laboratory temperature for 18 hours and then evaporated to dryness. Aqueous 10% w/v sodium carbonate solution (10 ml.) was added to the residue and the mixture was extracted with ethyl acetate. The extract was dried and evaporated to dryness and the residue was partially purified by flash chromatography on a silica gel (Merck 9385) column using a 60:35:20:1 v/v/v/v mixture of toluene, ethanol, ethyl acetate and aqueous ammonia solution (specific gravity 0.88) as eluent. The product was finally purified by preparative thin layer chromatography on silica gel plates (20 cm. x 20 cm., 2 mm thick) using a 3:1 v/v mixture of methylene chloride and methanol as developing solvent. There was thus obtained as an oil diethyl 4-o-chlorophenyl-1,4-dihydro-2-[{2-N-[2-(2-hydroxy-3-phenoxypropylamino)ethyl]carbamoyl}-acetamidomethyl]-6-methylpyridine-3,5-dicarboxylate, the structure of which was confirmed by elemental analysis, mass spectroscopy and proton magnetic resonance spectroscopy.

The carbamoylacetic acid used as starting material was obtained as follows:-

A solution of monoethyl malonyl chloride (0.79 g.) in diethyl ether (200 ml.) was added dropwise to a stirred solution of diethyl 2-aminomethyl-4-o-chlorophenyl-1,4-dihydro-6-methylpyridine-3,5-dicarboxylate (2.0 g.; described in United Kingdom Specification No. 1585978) and triethylamine (0.53 g.) in diethyl ether (400 ml.) at 0°C., and the mixture was stirred and allowed to warm up to laboratory temperature during 1 hour and was then washed successively with water, aqueous 10% w/v sodium bicarbonate solution and water,

dried and evaporated to dryness. The solid product was dissolved in methanol (20 ml.), aqueous 2N-sodium hydroxide solution (0.5 ml.) was added and the mixture was kept at laboratory temperature for 2 hours and then evaporated to dryness. Aqueous 20% w/v citric acid solution was added to the residue and the mixture was extracted with ethyl acetate. The extract was washed with water, dried and evaporated to dryness and the residue was crystallised from ethyl acetate. There was thus obtained N-(4-o-chlorophenyl-3,5-diethoxycarbonyl-1,4-dihydro-6-methylpyridine-2-ylmethyl)-2-carbamoylacetic acid, m.p. 160-170°C.

**Example 6**

A solution of 1-(2-aminoethylamino)-3-phenoxypropan-2-ol (0.21 g.) in methylene chloride (2 ml.) was added to a stirred solution of 2-(3,5-diethoxycarbonyl-1,4-dihydro-6-methyl-4-m-nitrophenylpyridin-2-ylmethoxy)acetic acid (0.448 g.), isobutyl chloroformate (0.13 ml.) and N-methylmorpholine (0.11 ml.) in methylene chloride (10 ml.) which had been stirred at -30°C. for 20 minutes, and the mixture was allowed to warm up to laboratory temperature and was stirred at that temperature for 2 hours, then washed with water, dried and evaporated to dryness. The residue was purified by flash chromatography on a silica gel (Merck 9385) column using a 1:19 v/v increasing to a 3:17 v/v mixture of methanol and methylene chloride as eluent. There was thus obtained as an oil diethyl 1,4-dihydro-2-{N-[2-(2-hydroxy-3-phenoxypropylamino)ethyl]-carbamoylmethoxymethyl}-6-methyl-4-m-nitrophenylpyridine-3,5-dicarboxylate hydrate, the structure of which was confirmed by elemental analysis, mass spectroscopy and proton magnetic resonance spectroscopy.

The acetic acid used as starting material was obtained as follows:-

A mixture of diethyl 1,4-dihydro-2-hydroxymethyl-6-methyl-4-m-nitrophenylpyridine-3,5-dicarboxylate (0.39 g.; described in United States Specification No. 4145423), t-butyl bromoacetate (0.2 g.), potassium carbonate (0.278 g.) and acetone (10 ml.) was heated under reflux for 5 hours. Further t-butyl bromoacetate (0.2 g.) and potassium carbonate (0.278 g.) were added and the mixture was heated under reflux for a further 3 hours and then evaporated to dryness. The residue was partitioned between methylene chloride (20 ml.) and water (20 ml.) and the organic layer was separated, washed with water, dried and evaporated to dryness. The residue was purified by flash chromatography on a silica gel (Merck 9385) column using a 4:1 v/v mixture of petroleum ether (b.p. 60-80°C.) and ethyl acetate as eluent.

A mixture of the t-butyl ester thus obtained (0.3 g.) and trifluoroacetic acid (3 ml.) was kept at laboratory temperature for 3 hours and the trifluoroacetic acid was removed by evaporation. There was thus obtained as residue 2-(3,5-diethoxycarbonyl-1,4-dihydro-6-methyl-4-m-nitrophenylpyridine-2-ylmethoxy)acetic acid, m.p. 134°C.

The process described in the first paragraph above was repeated using 1-(2-aminoethylamino)-3-(naphth-1-yloxy)propan-2-ol as starting material, and there was thus obtained as an oil diethyl 1,4-dihydro-2-[N-{2-[2-hydroxy-3-(naphth-1-yloxy)propylamino]ethyl}-carbamoylmethoxymethyl]-6-methyl-4-m-nitrophenylpyridine-3,5-dicarboxylate hemihydrate, the structure of which was confirmed by elemental analysis, mass spectroscopy and proton magnetic resonance spectroscopy.

Example 7

A solution of 1-(2-aminoethylamino)-3-phenoxypropan-2-ol (0.46 g.) in methylene chloride (5 ml.) was added to a stirred mixture of 2-(3,5-diethoxycarbonyl-1,4-dihydro-6-methyl-4-m-nitrophenylpyridin-2-yl)acetic acid (0.86 g.), dicyclohexylcarbodiimide (0.45 g.), 1-hydroxybenzotriazole (0.27 g.) and methylene chloride (20 ml.) which had been stirred at laboratory temperature for 30 minutes and the mixture was stirred at that temperature for 18 hours and then evaporated to dryness. The residue was partially purified by medium pressure liquid chromatography on a silica gel column using a 9:1 v/v mixture of methylene chloride and methanol as eluent, and was finally purified by preparative thin-layer chromatography on silica gel plates (20 cm. x 20 cm., 2 mm thick) using the same solvent mixture as developing solvent. There was thus obtained as an oil diethyl 1,4-dihydro-2-N-[2-(2-hydroxy-3-phenoxypropylamino)ethyl]carbamoylmethyl-6-methyl-4-m-nitrophenylpyridine-3,5-dicarboxylate hemihydrate, the structure of which was confirmed by elemental analysis, mass spectroscopy and proton magnetic resonance spectroscopy.

The acetic acid used as starting material was obtained as follows:-

A mixture of diethyl acetonedicarboxylate (11 g.), m-nitrobenzaldehyde (7.5 g.), ethyl 3-aminocrotonate (6.5 g.), acetic acid (1 ml.) and ethanol (200 ml.) was heated under reflux for 18 hours and then evaporated to dryness. A mixture of the residue (4.5 g.), ethanol (20 ml.) and aqueous 2N-sodium hydroxide solution (5 ml.) was kept at laboratory temperature for 2 hours and then evaporated to dryness and the residue was partitioned between water (50 ml.) and methylene chloride (50 ml.). The aqueous layer was separated,

neutralised with acetic acid and extracted with methylene chloride (50 ml.) and the extract was dried and evaporated to dryness. The residue was crystallised from toluene and there was thus obtained 2-(3,5-diethoxycarbonyl-1,4-dihydro-6-methyl-4-m-nitrophenylpyridin-2-yl)acetic acid, m.p. 115-117°C. (with decomposition).

**Example 8**

A mixture of 1-(2-aminoethylamino)-3-o-cyanophenoxypropan-2-ol (0.635 g.), diethyl 2-acrylamidomethyl-1,4-dihydro-6-methyl-4-m-nitrophenylpyridine-3,5-dicarboxylate (1.0 g.) and ethanol (15 ml.) was maintained at laboratory temperature for 7 days and then evaporated to dryness. The residue was purified by flash chromatography on a silica gel (Merck 9385) column using as eluent a 60:34:20 v/v/v mixture of toluene, ethanol and ethyl acetate to which was added an increasing amount of aqueous ammonia solution (specific gravity 0.88) up to a 60:35:20:4 v/v/v/v mixture. The residue was dissolved in ethanol and the solution was added to an excess of a solution of oxalic acid in ethanol. The mixture was filtered and the solid product was crystallised from aqueous ethanol. There was thus obtained diethyl 2-{3-[2-(3-o-cyanophenoxy-2-hydroxypropylamino)ethylamino]-propionamidomethyl}- 1,4-dihydro-6-methyl-4-m-nitrophenylpyridine-3,5-dicarboxylate dioxalate, m.p. 227-228°C.

The diethyl pyridine-3,5-dicarboxylate used as starting material was obtained as follows:-

A solution of acryloyl chloride (0.047 g.) in diethyl ether (15 ml.) was added dropwise to a stirred solution of diethyl 2-aminomethyl-1,4-dihydro-6-methyl-4-m-nitrophenylpyridine-3,5-dicarboxylate (0.2 g.;

described in United Kindom Specification No. 1585978) and triethylamine (0.052 g.) in diethyl ether (35 ml.) at 0°C., and the mixture was stirred for 30 minutes and then shaken with aqueous 2N-hydrochloric acid (10 ml.). The ethereal layer was separated, washed with saturated aqueous sodium chloride solution, dried and evaporated to dryness. The residue was purified by flash chromatography on a silica gel (Merck 9385) column using a 3:2 v/v mixture of petroleum ether (b.p. 60-80°C.) and ethyl acetate as eluent. There was thus obtained diethyl 2-acrylamidomethyl-1,4-dihydro-6-methyl-4-m-nitrophenylpyridine-3,5-dicarboxylate, m.p. 137-140°C.

What we claim is:-

1. A dihydropyridine of the formula:-

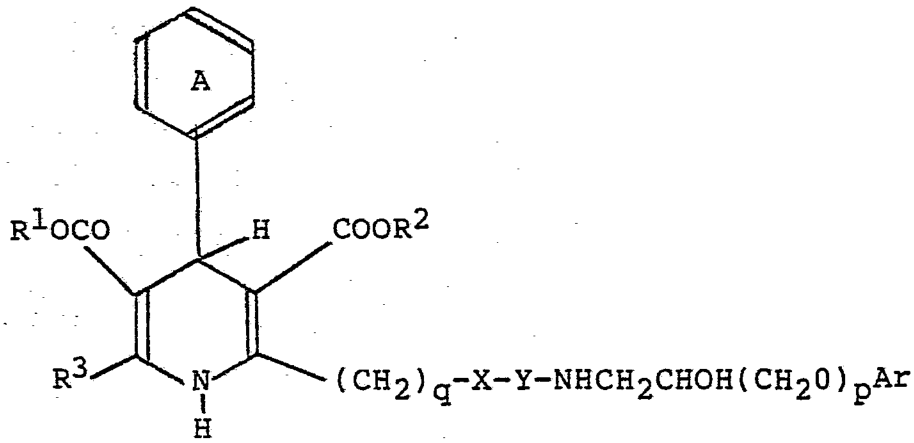

wherein $R^1$ and $R^2$, which may be the same or different; each is alkyl or alkoxyalkyl each of up to 6 carbon atoms:
wherein $R^3$ is alkyl of up to 6 carbon atoms;
wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus);
wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
or Ar is a 5- or 6-membered heterocyclic ring containing 1, 2 or 3 heteroatoms selected from oxygen, nitrogen and sulphur, which ring is saturated or unsaturated, which ring is unsubstituted or bears one or more substituents

selected from oxo, halogeno, trifluoromethyl, phenyl and morpholino, and alkyl and alkoxy each of up to 6 carbon atoms, and arylalkyl of up to 12 carbon atoms, and which ring may also be fused to a benzene ring, Ar being joined to the rest of the molecule either from the heterocyclic ring or from the fused benzene ring;

wherein p is 0 or 1;

wherein q is 1, 2, 3 or 4;

wherein X is -O-, -NH-, -NHCO- or -CONH-;

and wherein Y is straight-or branched-chain alkylene of 2 to 12 carbon atoms which may optionally be interrupted by one or two groups selected from oxygen (-O-), sulphur (-S-), imino and substituted imino (-$NR^4$ wherein $R^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;

or an acid-addition salt thereof.

2. A dihydropyridine derivative as claimed in claim 1 wherein $R^1$ and $R^2$, which may be the same or different, each is methyl or ethyl, wherein $R^3$ is methyl, wherein ring A is 2-chlorophenyl or 3-nitrophenyl, wherein Ar is phenyl which is unsubstituted or bears a chloro, cyano, methyl or methoxy substituent at the 2-position, or Ar is unsubstituted 1-naphthyl, 4-indolyl or 4-carbazolyl, wherein p and q are both 1, wherein X is -0-, -NHCO- or -CONH- and wherein Y is alkylene of 2 to 5 carbon atoms which may be interrupted by -0-, -NH-or -CONH-, or an acid-addition salt thereof.

3. A dihydropyridine as claimed in claim 2 wherein $R^1$ is methyl or ethyl, $R^2$ is ethyl, X is -0- and Y is alkylene of 2 to 4 carbon atoms which may be interrupted by -0-.

4. The compound ethyl 2-[2-(3-o-cyanophenoxy-2-hydroxypropylamino)ethoxy]methyl-1,4-dihydro-5-methoxycarbonyl-6-methyl-4-m-nitrophenylpyridine-3-carboxylate.

5. The compound ethyl 4-o-chlorophenyl-2-[2-(3-o-cyanophenoxy-2-hydroxypropylamino)ethoxy]methyl-1,4-dihydro-5-methoxycarbonyl-6-methylpyridine-3-carboxylate;
ethyl 2-{2-[3-(carbazol-4-yloxy)-2-hydroxypropylamino]ethoxy}methyl-1,4-dihydro-5-methoxycarbonyl-6-methyl-4-m-nitrophenylpyridine-3-carboxylate;
diethyl 2-[2-(3-o-cyanophenoxy-2-hydroxypropylamino)ethoxy]methyl-1,4-dihydro-6-methyl-4-m-nitrophenylpyridine-3,5-dicarboxylate;
ethyl 2-[3-(3-o-cyanophenoxy-2-hydroxypropylamino)propoxy]methyl-1,4-dihydro-5-methoxycarbonyl-6-methyl-4-m-nitrophenylpyridine-3-carboxylate;
ethyl 2-[4-(3-o-cyanophenoxy-2-hydroxypropylamino)-butoxy]methyl-1,4-dihydro-5-methoxycarbonyl-6-methyl-4-m-nitrophenylpyridine-3-carboxylate; or
ethyl 2-{2-[2-(3-o-cyanophenoxy-2-hydroxypropylamino)-ethoxy]ethoxy}methyl-1,4-dihydro-5-methoxycarbonyl-6-methyl-4-m-nitrophenylpyridine-3-carboxylate.

6. A process for the manufacture of a dihydropyridine, claimed in any of claims 1 to 5, which comprises:-

(a) the reaction of an amine of the formula:-

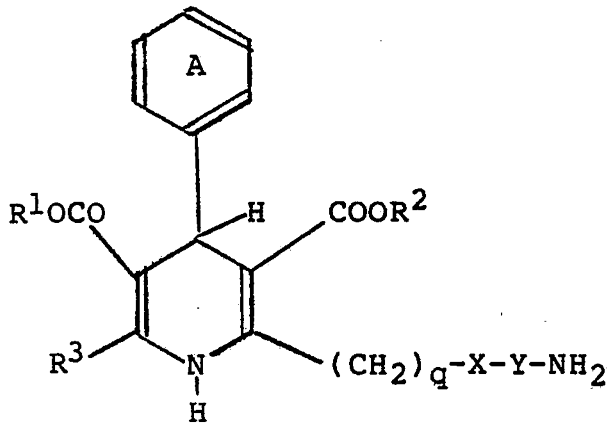

wherein A, $R^1$, $R^2$, $R^3$, q, X and Y have the meanings stated in any of claims 1 to 3, with an epoxide of the formula:-

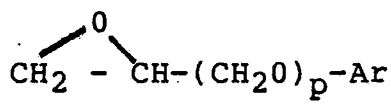

wherein Ar and p have the meanings stated in claim 1 or 2, or, when p is 0, with a haloketone of the formula

$$HalCH_2CO\text{-}Ar$$

wherein Ar has the meaning stated above and wherein Hal stands for a halogeno group, followed by reduction of the aminoketone thus obtained; or

(b) for the manufacture of a dihydropyridine wherein the group -Y- is alkylene interrupted by an amido group -CONH-, the reaction of an acid of the formula:-

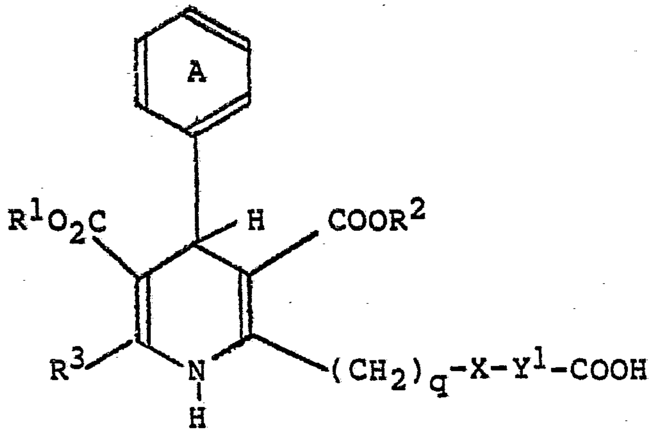

wherein A, $R^1$, $R^2$, $R^3$, q and X have the meanings stated above, or an activated derivative thereof, with an amine of the formula:-

$$H_2N-Y^2-NHCH_2CHOH(CH_2O)_pAr$$

wherein Ar and p have the meanings stated above, and wherein $Y^1$ and $Y^2$ are such that $-Y^1-CONH-Y^2-$ has the same meaning as stated above for Y; or

(c) the reaction of a compound of the formula:-

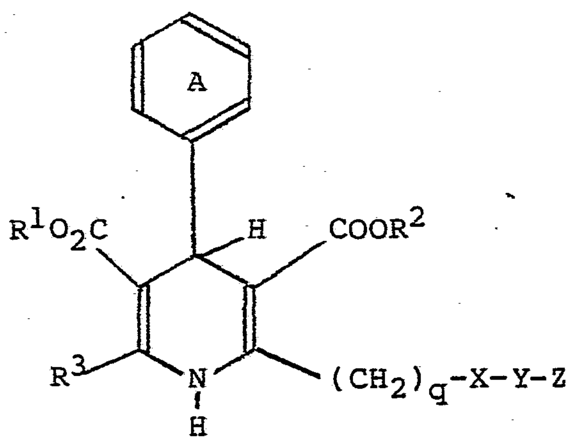

wherein A, $R^1$, $R^2$, $R^3$, q, X and Y have the meanings stated above and wherein Z stands for a displaceable group, with an amine of the formula:-

$$H_2NCH_2CHOH(CH_2O)_p-Ar$$

wherein Ar and p have the meanings stated above; or (d) the rection of an aldehyde of the formula

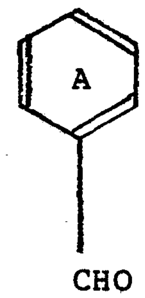

wherein A has the meaning stated above, an aminocrotonate of the formula

$$R^3-\overset{\displaystyle NH_2}{\overset{|}{C}}=CH-CO_2R^1$$

wherein $R^1$ and $R^3$ have the meanings stated above and a ketoacid derivative of the formula

$$R^2O_2CCH_2CO(CH_2)_q-X-Y-NHCH_2CHOH(CH_2O)_p-Ar$$

wherein Ar, p, q, $R^2$, X and Y have the meanings stated above.

7. A pharmaceutical composition comprising as active ingredient at least one dihydropyridine, claimed in any of claims 1 to 5, or an acid-addition salt thereof, in association with a pharmaceutically-acceptable diluent or carrier therefor.

8. A composition as claimed in claim 7 which is a tablet, capsule, aqueous or oily solution or suspension, dispersible powder, spray or aerosol formulation.

9. A composition as claimed in claim 7 or 8 which contains, in addition to the dihydropyridine, one or more drugs selected from sedatives, vasodilators, diuretics, other antihypertensive agents, cardiac membrane stabilising agents, agents used in the treatment of Parkinson's disease and other tremors, cardiotonic agents, and alpha-adrenergic blocking agents.

10. The use of a compound, claimed in any of claims 1 to 5, for the manufacture of a medicament for producing an antihypertensive effect in a warm-blooded animal.

REGINALD PETER SLATCHER

AUTHORISED REPRESENTAT
General Authorisation No.

SE03
PS33369
RPS/MJW: 15 JAN 86

CLAIMS FOR AUSTRIA

What we claim is:-

1. A process for the manufacture of a dihydropyridine of the formula:-

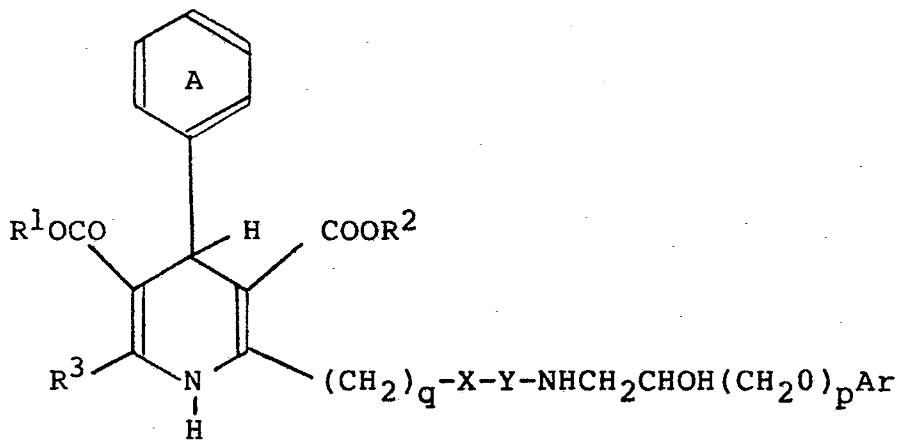

wherein $R^1$ and $R^2$, which may be the same or different, each is alkyl or alkoxyalkyl each of up to 6 carbon atoms:
wherein $R^3$ is alkyl of up to 6 carbon atoms;
wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus);
wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
or Ar is a 5- or 6-membered heterocyclic ring containing

1, 2 or 3 heteroatoms selected from oxygen, nitrogen and sulphur, which ring is saturated or unsaturated, which ring is unsubstituted or bears one or more substituents selected from oxo, halogeno, trifluoromethyl, phenyl and morpholino, and alkyl and alkoxy each of up to 6 carbon atoms, and arylalkyl of up to 12 carbon atoms, and which ring may also be fused to a benzene ring, Ar being joined to the rest of the molecule either from the heterocyclic ring or from the fused benzene ring;
wherein p is 0 or 1;
wherein q is 1, 2, 3 or 4;
wherein X is -O-, -NH-, -NHCO- or -CONH-;
and wherein Y is straight-or branched-chain alkylene of 2 to 12 carbon atoms which may optionally be interrupted by one or two groups selected from oxygen (-O-), sulphur (-S-), imino and substituted imino (-NR$^4$ wherein R$^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof, characterised by:-
(a) the reaction of an amine of the formula:-

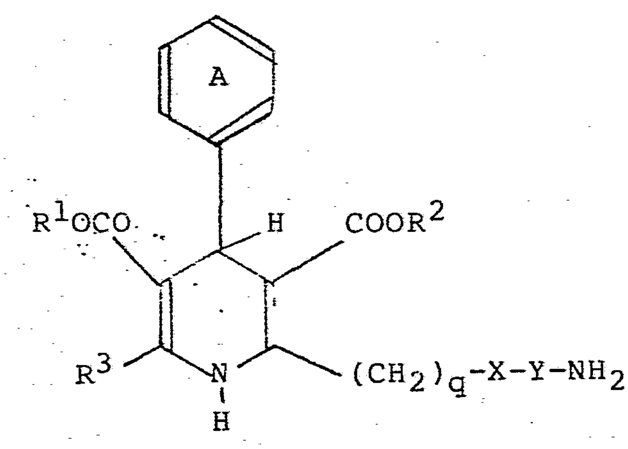

wherein A, $R^1$, $R^2$, $R^3$, q, X and Y have the meanings stated above, with an epoxide of the formula:-

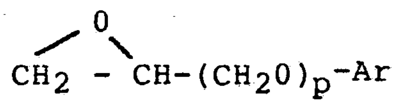

wherein Ar and p have the meanings stated above or, when p is 0, with a haloketone of the formula

$$HalCH_2CO\text{-}Ar$$

wherein Ar has the meaning stated above and wherein Hal stands for a halogeno group, followed by reduction of the aminoketone thus obtained; or

(b) for the manufacture of a dihydropyridine wherein the group -Y- is alkylene interrupted by an amido group -CONH-, the reaction of an acid of the formula:-

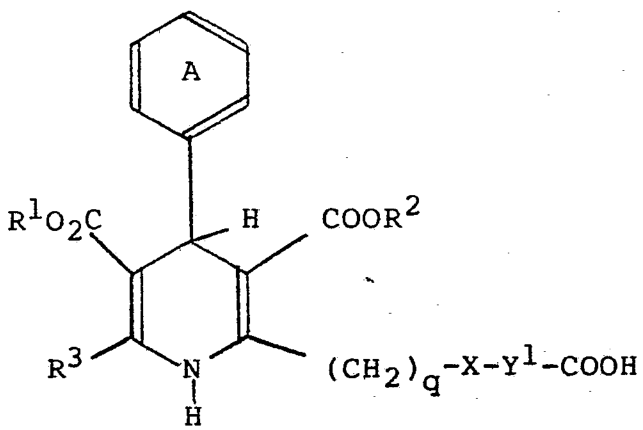

wherein A, $R^1$, $R^2$, $R^3$, q and X have the meanings stated above, or an activated derivative thereof, with an amine of the formula:-

$$H_2N\text{-}Y^2\text{-}NHCH_2CHOH(CH_2O)_pAr$$

wherein Ar and p have the meanings stated above, and wherein $Y^1$ and $Y^2$ are such that $-Y^1-CONH-Y^2-$ has the same meaning as stated above for Y; or

(c) the reaction of a compound of the formula:-

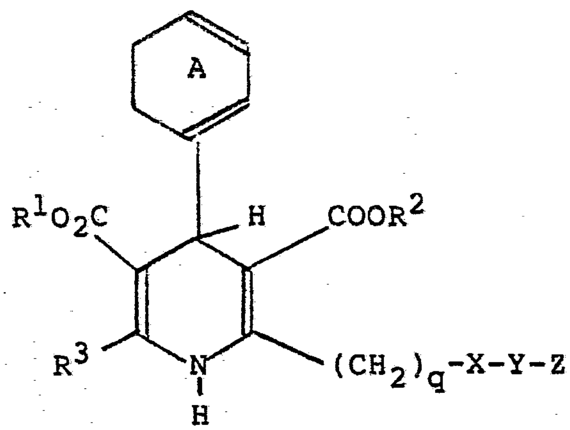

wherein A, $R^1$, $R^2$, $R^3$, q, X and Y have the meanings stated above and wherein Z stands for a displaceable group, with an amine of the formula:-

$$H_2NCH_2CHOH(CH_2O)_p-Ar$$

wherein Ar and p have the meanings stated above; or

(d) the rection of an aldehyde of the formula

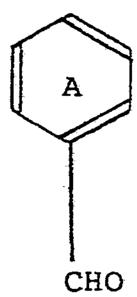

wherein A has the meaning stated above, an aminocrotonate of the formula

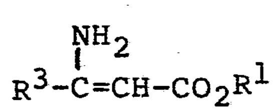

wherein $R^1$ and $R^3$ have the meanings stated above and a ketoacid derivative of the formula

$$R^2O_2CCH_2CO(CH_2)_q\text{-X-Y-NHCH}_2\text{CHOH}(CH_2O)_p\text{-Ar}$$

wherein Ar, p, q, $R^2$, X and Y have the meanings stated above.

2. A process as claimed in claim 1 wherein in the starting materials $R^1$ and $R^2$, which may be the same or different, each is methyl or ethyl, $R^3$ is methyl, ring A is 2-chlorophenyl or 3-nitrophenyl, Ar is phenyl which is unsubstituted or bears a chloro, cyano, methyl or methoxy substituent at the 2-position, or Ar is unsubstituted 1-naphthyl, 4-indolyl or 4-carbazolyl, p and q are both 1, X is -O-, -NHCO-or -CONH-and Y is alkylene of 2 to 5 carbon atoms which may be interrupted by -O-, -NH-or -CONH-.

3. A process as claimed in claim 2 wherein $R^1$ is methyl or ethyl, $R^2$ is ethyl, X is -O- and Y is alkylene of 2 to 4 carbon atoms which may be interrupted by -O-.

REGINALD PETER SLATCHER

AUTHORISED REPRESENTA[illegible]
General Authorisation No.

PS33368AT
SE03
RPS/MJW: 6 Feb 86

European Patent Office

# EUROPEAN SEARCH REPORT

0194752

Application number

EP 86 30 0887

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | GB-A-1 409 865 (SCIENCE UNION) * Example 10; pages 6,7; claims * | 1,6-10 | C 07 D 211/90<br>C 07 D 401/12<br>C 07 D 413/04<br>A 61 K 31/44 |
| A | GB-A-1 425 059 (BAYER) * Examples 6,7; page 15; claims * | 1,6-10 | |
| A | GB-A-1 552 911 (FUJISAWA) * Example 5; page 32; claims * | 1,6-10 | |
| A | EP-A-0 002 231 (BAYER) * Example 14; claims * | 1,6-10 | |
| D,A | JOURNAL OF MEDICINAL CHEMISTRY, vol. 24, no. 5, May 1981, pages 628-631, American Chemical Society, Washington, US; J.J. BALDWIN et al.: "Approaches to vasodilating/bêta-adrenergic blocking agents: examples of the dihydrolutidine type" * Page 629, compounds 4a,b,c; page 631, expermental section; pages 630-631, discussion * | 1,6-10 | TECHNICAL FIELDS SEARCHED (Int. Cl.4)<br>C 07 D 211<br>C 07 D 401<br>C 07 D 413<br>A 61 K 31 |

The present search report has been drawn up for all claims

| Place of search | Date of completion of the search | Examiner |
|---|---|---|
| THE HAGUE | 28-05-1986 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82